# EUROPEAN PATENT SPECIFICATION

(11) **EP 2 648 600 B1**
(45) Date of publication and mention of the grant of the patent: **20.01.2016**
(21) Application number: 12702116.0
(22) Date of filing: 20.01.2012
(51) Int. Cl.: A61B 1/00, A61B 5/00, A61B 3/10, A61B 1/313, A61B 18/20, A61B 18/22, A61F 9/008

(54) **COMBINED SURGICAL ENDOPROBE FOR OPTICAL COHERENCE TOMOGRAPHY, ILLUMINATION OR PHOTOCOAGULATION**
KOMBINIERTE CHIRURGISCHE ENDOSONDE ZUR OPTISCHEN KOHÄRENZTOMOGRAPHIE, BELEUCHTUNG ODER PHOTOKOAGULATION
ENDOSONDE CHIRURGICALE COMBINÉE POUR TOMOGRAPHIE PAR COHÉRENCE OPTIQUE (OCT), ÉCLAIRAGE OU PHOTOCOAGULATION

(30) Priority: 21.01.2011 US 201161435031 P
(43) Date of publication of application: 16.10.2013
(73) Proprietor: Alcon Research, Ltd., Fort Worth, TX 76134-2099 (US)
(72) Inventor: YADLOWSKY, Michael J., Sunnyvale CA 94087 (US); PAPAC, Michael James, North Tustin CA 92705 (US); HUCULAK, John Christopher, Mission Viejo CA 92692 (US)
(74) Representative: Hanratty, Catherine
(86) International application number: PCT/US2012/021990
(87) International publication number: WO 2012/100138

(56) References cited:
- US-A- 6 004 314
- US-A1- 2010 228 119
- US-B1- 6 485 413

## Description

### Background

### 1. Field of the Invention

Embodiments described herein relate to the field of microsurgical probes. More particularly, embodiments described herein are related to the field of surgical endoprobes combining optical coherence tomography with illumination or photocoagulation.

### 2. Description of Related Art

The field of microsurgical procedures is evolving rapidly. Typically, these procedures involve the use of probes that are capable of reaching the tissue that is being treated or diagnosed. Such procedures make use of endoscopic surgical instruments having a probe coupled to a controller device in a remote console. Current state of the art probes are quite complex in operation, often times requiring moving parts that are operated using complex mechanical systems. In many cases, an electrical motor is included in the design of the probe. Most of the prior art devices have a cost that makes them difficult to discard after one or only a few surgical procedures. Furthermore, the complexity of prior art devices leads generally to probes having cross sections of several millimeters. These probes are of little practical use for ophthalmic microsurgical techniques. In ophthalmic surgery, dimensions of one (1) mm or less are preferred, to access areas typically involved without damaging unrelated tissue.

Scanning mechanisms that allow time-dependent direction of light for diagnostic or therapeutic purposes have been used in endoscopic surgical instruments. These instruments typically use probes that provide imaging, treatment, or both, over an extended area of tissue without requiring motion of the endoscope relative to its surroundings. However, there are typically multiple probes for each function, and different light sources are used for different applications.

Therefore, there remains a need for surgical endoprobes combining different functions in a synergistic manner.

### Summary

The object of the present invention is defined in claim 1. According to particular embodiments of the present invention, a surgical system includes a surgical laser source operable to emit a surgical laser beam and an OCT engine operable to emit an OCT beam. The surgical system also includes an endoprobe optically coupled to the surgical laser source and the OCT engine. The endoprobe includes an OCT fiber for transmitting the OCT beam, a surgical laser fiber for transmitting the surgical laser beam, and scanning optics optically coupled to the OCT fiber and the surgical laser fiber, the scanning optics configured to simultaneously scan both the OCT beam and the surgical laser beam. The surgical system further includes a processor programmed to control the scanning optics to scan the OCT beam and the surgical laser beam over a targeted tissue area and to detect an OCT signal from the targeted tissue area.

Various embodiments of the present invention will also extend to methods of operation consistent with the description provided and the steps performed by various elements of the surgical system. Likewise, embodiments of the present invention may extend to software embodied in a computer-readable medium used to control a surgical system in the manner described. This would also extend to any suitable variations apparent to one skilled in the art that would make similar modifications to the methods and software apparent as well.

These and other embodiments of the present invention will be described in further detail below with reference to the following drawings.

### Brief Description of the Drawings

FIG. 1 is a block diagram of a surgical system according to a particular embodiment of the present invention;
FIG. 2 illustrates an endoprobe according to a particular embodiment of the present invention;
FIG. 3 illustrates a distal end of an endoprobe according to a particular embodiment of the present invention; and
FIG. 4 illustrates a distal end of an endoprobe according to another particular embodiment of the present invention.

In the figures, elements having the same reference number have the same or similar functions.

### Detailed Description

Various embodiments of the present invention provide an endoprobe with OCT scanning combined with surgical laser applications and/or illumination. The probe may be a hand-held probe, for direct manipulation by specialized personnel. In some embodiments, the probe may be designed to be controlled by a robotic arm or a computer-controlled device. Probes have a proximal end close to the operation controller (be it a specialist or a device), and a distal end, close to or in contact with the tissue. Probes according to embodiments disclosed herein may have small dimensions, be easy to manipulate from a proximal end, and minimally invasive to the surrounding tissue. In the distal end, the probe ends with a tip, from where the probe performs certain action on a target tissue located in the vicinity of the tip. For example, the probe may deliver light from its tip, and receive light reflected or scattered from the tissue, coupled through the tip. The tip of the probe may include movable elements that enable the tip to perform its action.

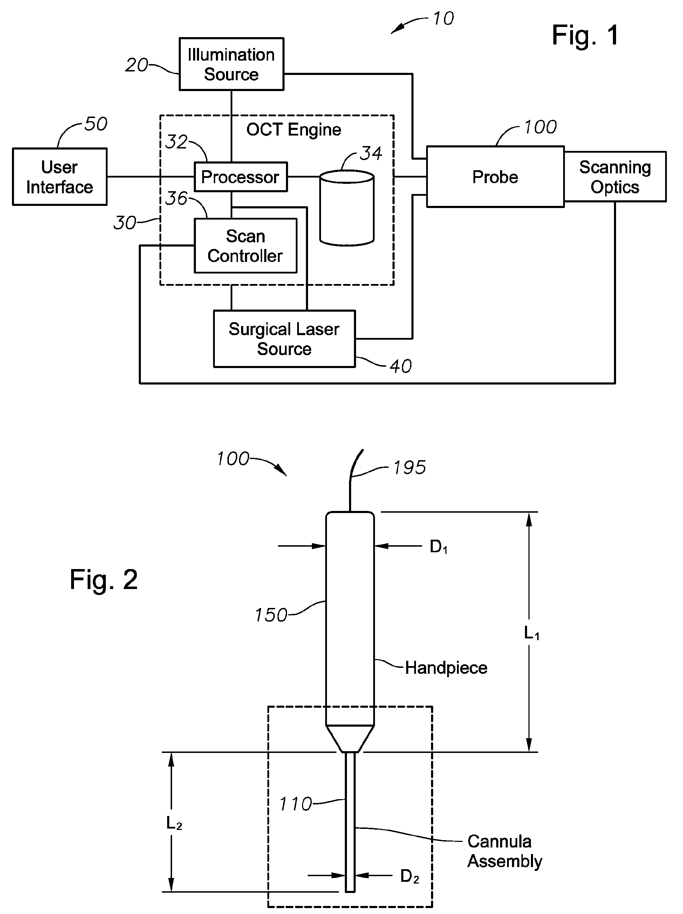

FIG. 1 is a block diagram of a surgical system 10 according to a particular embodiment of the present invention. In the depicted embodiment, the surgical system 10 includes an illumination source 20 for producing visible light suitable for visualization of a surgical field and an OCT engine 30. The surgical laser source 40 provides laser energy having suitable properties to effect a modification of targeted tissue, such as photocoagulation of retinal tissue. While these elements are discussed in detail below, it will be understood that surgical system 10 may also include other surgical light sources, such as a laser source for photocoagulation, trabeculectomy, or other surgical applications. The following description may be suitably adapted to include a variety of light delivery applications known in the art. The illumination source 20, the OCT engine 30, and the surgical laser source 40 are coupled to a surgical probe 100 using suitable coupling optics that may be selected based on the output beam having desired properties of the light being delivered for a particular application, such as energy, wavelength, or numerical aperture. The surgical system 10 further includes a user interface 50 that allows a user to control the operation of the surgical system 10, which may comprise any suitable input or output devices including but not limited to a keyboard, a hand-held control, a mouse, a touch screen, a footswitch, a microphone for voice commands, or any of the numerous such devices known in conventional surgical systems.

The illumination source 20 may be any of the numerous surgical illumination sources, such as a xenon lamp, a collection of light emitting diodes, a laser, or any other suitable light source for generating light falling within the visible light spectrum. The OCT engine 30 is an interferometry apparatus for measuring the interference between a reference beam generated using the surgical light and light returning from the tissue illuminated by the surgical light. In particular embodiments, the OCT engine 30 may include a spectrometer-based interferometer, also known as "spectral domain OCT." This refers to an OCT system that uses a relatively broad spectral range of light and measures interference of discrete wavelengths within the spectral band to reconstruct information about the target tissue.

The OCT engine 30 also includes a processor 32, which may be one or more suitable electronic components for processing information, including but not limited to a microprocessor, microcontroller, application-specific integrated circuit (ASIC), or other programmable device. The processor 32 processes information about the interference produced by light reflected from the tissue to generate a mathematical representation of the scanned tissue, which may in turn be used to produce an electronic image of the tissue. The OCT engine 30 also includes a memory 34, which may be any suitable form of information storage including electronic, magnetic, or optical storage that may be either volatile or non-volatile. Finally, the OCT engine 30 includes a scan controller 36. The scan controller 36 may be any suitable combination or hardware, software, and/or firmware and mechanical components, which may include processor 32 and memory 34, suitable for controlling the movement of optical components to redirect the surgical light used by the OCT engine 30. For example, in embodiments where a probe 100 includes scanning optics for the OCT beam, the scan controller 66 may be connected to the scanning optics in order to control the scanning mechanism.

In one example of OCT imaging techniques, a light beam having a coherence length may be directed to a certain spot in the target tissue by using a probe. The coherence length provides a resolution depth, which when varied at the distal end of the probe may be de-convolved to produce an in-depth image of the illuminated portion of the tissue (A-scan). A 2-dimensional tissue image may be obtained through a B-scan. In some embodiments, B-scans are straight lines along a cross-section of the tissue. Furthermore, by performing repeated B-scans along different lines in the tissue, a 3D rendition of the tissue may be provided. In some embodiments, the B-scans may be a set of lines having the same length and arranged in a radius from a common crossing point. Thus, the plurality of B-scans provides an image of a circular area in the tissue, having a depth.

In some embodiments, OCT techniques use forward-directed scan procedures. In this case, optical illumination takes place in the forward direction of the probe longitudinal axis. In forward-directed scans, the target tissue may be ahead of the probe in a plane perpendicular to the probe longitudinal axis. Thus, light traveling from the tip of the probe to the tissue, and back from the tissue into the probe may travel in a direction substantially parallel to the probe longitudinal axis. In some embodiments using forward-directed scans, the target tissue may be approximately perpendicular to the probe longitudinal axis, but not exactly. Furthermore, in some embodiments light traveling to and from the target tissue from and into the probe may not be parallel to the probe longitudinal axis, but form a symmetric pattern about the probe longitudinal axis. For example, light illuminating the target tissue in a forward-directed scan may form a solid cone or a portion thereof about the probe longitudinal axis. Likewise, light collected by an endoprobe in a forward-directed scan may come from target tissue in a 3D region including a portion of a cone section around the probe longitudinal axis.

FIG. 2 shows microsurgical endoprobe 100 that includes a cannula assembly 110 and a hand-piece housing 150. A cannula assembly 110 includes the distal end of endoprobe 100 which may be elongated along the probe longitudinal axis and have a limited cross-section. For example, in some embodiments cannula assembly 110 may be about 0.5 mm in diameter (D₂) while hand-piece 150 may have a substantially cylindrical shape of several mm in diameter (D₁) such as 12-18 mm. A coupling cable 195 includes light guides carrying light from the coupling optics 50 of the broadband light source 20. In alternative embodiments, separate probes 100 could be coupled to the common light source, or both surgical light and illumination light could be coupled into a common light guide.

In some embodiments, assembly 110 may be in contact with tissue, including target tissue for the microsurgical procedure. Thus, assembly 110 may be coated with materials that prevent infection or contamination of the tissue. Furthermore, surgical procedures and protocols may establish hygienic standards for assembly 110.

For example, it may be desirable that assembly 110 be disposed of after used once. In some situations, assembly 110 may be disposed of at least every time the procedure is performed on a different patient, or in a different part of the body.

Hand-piece housing 150 may be closer to the proximal end of the probe, and may have a larger cross section as compared to element 110. Element 150 may be adapted for manual operation of endoprobe 100, according to some embodiments. Element 150 may be adapted for robotic operation or for holding by an automated device, or a remotely operated device. While assembly 110 may be in contact with living tissue, element 150 may not be in direct contact with living tissue. Thus, even though element 150 may comply with hygienic standards, these may be somewhat relaxed as compared to those used for assembly 110. For example, element 150 may include parts and components of endoprobe 100 that may be used repeatedly before disposal.

Thus, some embodiments of endoprobe 100 as disclosed herein may include complex components in element 150, and less expensive, replaceable components may be included in assembly 110. Some embodiments may have a removable element 110 which is disposable, while hand-piece 150 may be used more than once. Hand-piece 150 may be sealed hermetically, in order to avoid contamination of the tissue with particulates or fumes emanating from internal elements in hand-piece 150. In some embodiments, cannula assembly 110 may be fixed to hand-piece 150 by an adhesive bonding. According to other embodiments, assembly 110 may be removable from hand-piece 150, to allow easy replacement of endoprobe 100 for repeated procedures. Some embodiments consistent with FIG. 2 may have a disposable element 150 and a disposable assembly 110.

In some embodiments, an OCT technique may use side imaging. For example, in side imaging the target tissue may be parallel to a plane containing the probe longitudinal axis. In a situation like this, it may be desirable to move the illumination spot in a circular trajectory around the probe longitudinal axis, to create a closed-loop image of the target tissue. Such a situation may arise in microsurgery involving endovascular procedures. For example, in coronary angiography the interior wall of the coronary artery may be fully scanned in cylindrical sections along the arterial lumen using embodiments described herein.

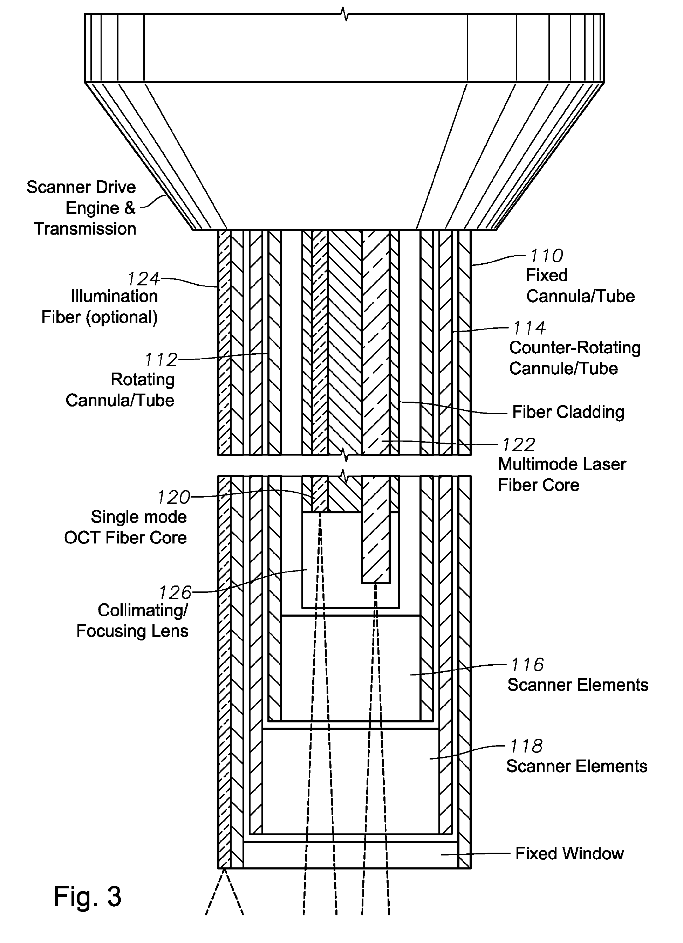

FIG. 3 is a schematic illustrating particular features of the cannula assembly 110 of an example endoprobe 100 according to particular embodiments of the present invention. In the depicted embodiment, the cannula 110 includes two counter-rotating internal cannulae 112 and 114 having corresponding scanner elements 116 and 118, which may be gradient index (GRIN) lenses. The scanner elements 116 and 118 rotate with respect to one another to scan a beam. The operation of these elements is described in greater detail in the co-pending application entitled "Counter-rotating Ophthalmic Scanner Drive Mechanism," filed on January 21, 2011, as application serial no. 61/434,942. More generally, any collection of movable optical elements suitable for scanning a light beam, generally referred to as "scanning optics," could be employed.

The cannula 110 also encloses an OCT fiber 120, a surgical laser fiber 122, and an illumination fiber 124 within a wall of the cannula 110. The illumination fiber 124 delivers light in the visible range from the illumination source 20, while the OCT fiber 120 delivers light within suitable spectrum from the OCT engine 30 and returns light reflected from tissue for interferometry measurements. The surgical laser fiber 122 similarly delivers laser energy from the surgical laser source 40. In the depicted embodiment, the OCT fiber 120 and the surgical laser fiber 122 can use a common cladding, but a stacked arrangement of separate fibers could also be used.

Because of the wavelength requirements required for OCT measurements, a single-mode fiber may be suitable for the OCT fiber 120, while the surgical laser fiber 122 may be multimode in order to deliver sufficient energy for tissue modification with relatively high efficiency. A collimating and/or focusing lens 126 may be useful for assuring that the light emitted from the OCT fiber 120 and the surgical laser fiber 122 are focused at a common plane, so that the OCT scan follows in close proximity to the surgical laser beam. As shown, the OCT and surgical laser beams can then be co-scanned by the scanner elements 116 and 118. This advantageously allows the modification of tissue by the surgical laser to be monitored.

Separate optical elements may also be used in place of lens 126 and/or scanner elements 116 and 118 for each fiber, allowing the beams to be scanned in different ranges or at different rates. In particular embodiments, lens 126 associated with the surgical laser fiber 122 produces a multiple spot pattern from the surgical laser beam using any suitable optical configuration, which may in turn be scanned by the scanner elements 116 and 118. In other embodiments, the surgical laser source 40 may use one or more optical elements to emit multiple beams coupled into multiple surgical laser fibers 122 that produce multiple surgical laser spots.

The processor 32 of the OCT engine 30 may be programmed to detect tissue configurations characteristic of particular conditions, such as when the tissue modification has been successfully achieved in the target region. The processor 32 may also be programmed to detect when excessive energy has been delivered, such as when tissue has been burned, and to take remedial action. For example, the surgical laser source 40 could be signaled to shut off, or the scanner elements 116 and 118 could be controlled to move the beam more quickly to a new area of targeted tissue. In general, the scanning of the photocoagulation beam can be automated based on monitoring of the OCT signal to facilitate effective and uniform modification of the issue.

While the example of photocoagulation has been provided, any other application of optical energy for tissue modification could also be similarly controlled. Furthermore, although the configuration with processor 32 of the OCT engine 30 has been given as an example, any suitable arrangement of control electronics for a surgical system, including any number of separate processors for controlling various subsystems of the surgical system 10 could be used as well. Hence, the term "processor" can refer generally to any component or collection of components, including any suitable volatile or non-volatile memory for storing information, that are capable of directing the operations of various elements of a surgical system 10.

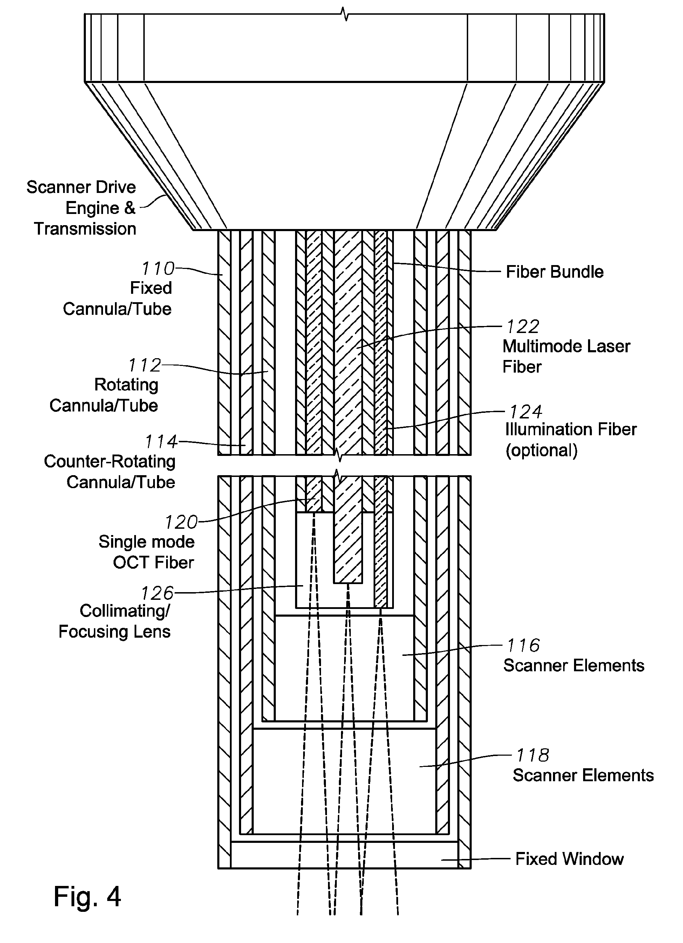

FIG. 4 illustrates a different embodiment of the cannula 110 of the probe 100. In the embodiment of FIG. 4, light from the illumination fiber 124 can also be scanned. An advantage of scanning the illumination beam, such as in the embodiment of FIG. 4, is that the beam can be swept to cover a larger area, effectively increasing the numerical aperature of the illumination light and directly linking the visualization of tissue to the scanning of the surgical laser beam and/or the OCT beam. A moderately high scan rate useful for OCT, such as 60 Hz, is typically also sufficient to make the illumination appear constant and uniform within the field of view.

Various embodiments can also advantageously adjust the duty cycle of the illumination source 20, the OCT engine 30, and/or the surgical laser source 40 in order to produce desired scanning patterns. For example, the surgical laser source 40 could be activated at selected points while the OCT beam and illumination beams are being scanned to produce a desired laser pattern on the target tissue. Likewise, the relative size of the spot pattern for the surgical laser to the illumination field could also be selected.

In particular embodiments, the scan pattern may also be programmable or selectable among a number of options using the user interface 50. Thus, for example, a surgeon who wished to adjust the size of the spot pattern relative to the field of illumination or to widen the field of illumination could provide suitable input to do so. In one such example, the spot pattern and illumination field could be displayed on a touch screen, and the surgeon could drag his finger across the touch screen to reshape or resize elements of the pattern. Constraints could also be set on the possible patterns in order to prevent situations such spots from being too close together so as to increase the likelihood of tissue damage. The processor 32 can also be programmed to determine the expected rate of tissue modification and to adjust the dwell time of particular spots based on the duty cycle and/or scan rate, and the laser surgery can also be monitored and controlled based on OCT feedback. A number of other possible customizations based on surgical needs, suitable illumination, and safety considerations will be apparent to one skilled in the art.

Although the foregoing description has focused on the surgical system and probe apparatus, it should be understood that various embodiments of the present invention will also extend to methods of operation consistent with the description provided above and the steps performed by various elements of the surgical system. Likewise, embodiments of the present invention may extend to software embodied in a computer-readable medium used to control a surgical system in the manner described. This would also extend to any suitable variations apparent to one skilled in the art that would make similar modifications to the methods and software apparent as well.

Various embodiments of the present invention provide an endoprobe with OCT scanning combined with surgical laser applications and/or illumination. Embodiments of the invention described above are exemplary only. One skilled in the art may recognize various alternative embodiments from those specifically disclosed. Those alternative embodiments are also intended to be within the scope of this disclosure. As such, the invention is limited only by the following claims.

## Claims

1. A surgical system (10), comprising:
a surgical laser source (40) operable to emit a surgical laser comprising at one optical element;
an OCT engine (30) operable to emit an OCT beam;
an endoprobe (100) optically coupled to the surgical laser source (40) and the OCT engine (30), the endoprobe comprising:
an OCT fiber (120) for transmitting the OCT beam;
a surgical laser fiber (122) for transmitting the surgical laser beam; the surgical laser beam passing through the at least one optical element of the surgical laser source;
a cannula (110) comprising a first and a second counter-rotating internal cannulae (112,114), respectively having first scanning optics (116) and second scanning optics, said cannula enclosing the OCT fiber (120) and the surgical laser fiber (122), optically coupled to the first and second scanning optics such that the OCT beam and the surgical laser beam each pass through the first and second scanning optics, counter rotation of the first and second scanning optics causing simultaneously scanning of both the OCT beam and the surgical laser beam; and
a processor (32) programmed to control the scanning optics to scan the OCT beam and the surgical laser beam over a targeted tissue area and to detect an OCT signal from the targeted tissue area,
**characterized in that** the at least one optical element is configured to produce multiple spots from the surgical laser beam.

2. The surgical system of Claim 1, wherein the processor (32) is programed to determine that the targeted tissue area has been successfully modified by the surgical laser beam and to scan the surgical laser beam and the OCT beam to another tissue are in response.

3. The surgical system of Claim 1, wherein the processor (32) is programmed to determine that the targeted tissue area has been damaged and to initiate a remedial action in response.

4. The surgical system of Claim 3, wherein the remedial action is to shut off the surgical laser source.

5. The surgical system of Claim 1, wherein the surgical system further comprises an illumination source (20) optically coupled to the endoprobe (100), and the endoprobe further comprises an illumination fiber (124).

6. The surgical system of Claim 5, wherein the illumination fiber (124) is optically coupled to the scanning optics.

7. The surgical system of Claim 6, wherein the surgical system (10) further comprises a user interface (50), and the processor (32) is further programmed to change an illumination scan pattern in response to input from the user interface.

8. The surgical system of Claim 1, wherein the processor (32) is further programmed to control a duty cycle of the surgical laser source to produce a selected spot pattern for the surgical laser beam.

9. The surgical system of Claim 8, wherein the surgical system (10) further comprises a user interface (50), and the processor (32) is further programmed to change the selected spot pattern in response to input from the user interface.

10. The surgical system of Claim 1, wherein the surgical laser (40) source emits a plurality of surgical laser beams, and the endoprobe comprises a plurality of surgical laser fibers.

## Patentansprüche

1. Ein chirurgisches System (10), aufweisend:
eine chirurgische Laserquelle (40), die zum Emittieren eines chirurgischen Lasers betreibbar ist, mit mindestens einem optischen Element;
eine OCT-Maschine (30), die zum Emittieren eines OCT-Strahls betreibbar ist;
eine Endosonde (100), die optisch an die chirurgische Laserquelle (40) und die OCT-Maschine (60) gekoppelt ist, wobei die Endosonde aufweist:
eine OCT-Faser (120) zum Übertragen des OCT-Strahls;
eine chirurgische Laserfaser (122) zum Übertragen des chirurgischen Laserstrahls; wobei der chirurgische Laserstrahl durch das mindestens eine optische Element der chirurgischen Laserquelle hindurch läuft;
eine Kanüle (110) aufweisend eine erste und eine zweite einander gegenrotierende interne Kanülen (112, 114), die jeweils erste Scanoptiken (116) und zweite Scanoptiken aufweisen, wobei die besagte Kanüle die OCT-Faser (120) und die chirurgische Laserfaser (122) umschließt, optisch gekoppelt an die erste und zweite Scanoptiken, derart, dass der OCT-Strahl und der chirurgische Laserstrahl jeweils durch die ersten und zweiten Scanoptiken hindurchtreten, wobei eine Gegenrotation der ersten und zweiten Scanoptiken ein gleichzeitiges Scannen von sowohl dem OCT-Strahl als auch dem chirurgischen Laserstrahl verursacht; und
einen Prozessor (32), der zum Steuern der Scanoptiken programmiert ist, um den OCT-Strahl und den chirurgischen Laserstrahl über ein Zielgewebebereich zu steuern und ein OCT-Signal von dem Ziel-Gewebebereich zu detektieren,
**dadurch gekennzeichnet, dass** mindestens ein optisches Element zum Erzeugen multipler Spots von dem chirurgischen Laserstrahl konfiguriert ist.

2. Chirurgisches System nach Anspruch 1, wobei der Prozessor (32) programmiert ist, zu bestimmen, dass der Zielgewebebereich durch den chirurgischen Laserstrahl erfolgreich modifiziert worden ist, und in Antwort hierauf den chirurgischen Laserstrahl und den OCT-Strahl an ein anderes Gewebe zu scannen.

3. Chirurgisches System nach Anspruch 1, wobei der Prozessor (32) programmiert ist, um zu bestimmen, dass der Zielgewebebereich beschädigt worden ist, und um in Antwort eine Abhilfemaßnahme hierzu zu initiieren.

4. Chirurgisches System nach Anspruch 3, wobei die Abhilfemaßnahme darin liegt, die chirurgische Laserquelle abzuschalten.

5. Chirurgisches System nach Anspruch 1, wobei das chirurgische System ferner eine Beleuchtungsquelle (20) aufweist, die optisch an die Endosonde (100) angeschlossen ist, und die Endosonde ferner eine Beleuchtungsfaser (124) aufweist.

6. Chirurgisches System nach Anspruch 5, wobei die Beleuchtungsfaser (124) optisch an die Scanoptiken gekoppelt ist.

7. Chirurgisches System nach Anspruch 6, wobei das chirurgische System (10) eine Anwenderschnittstelle (50) aufweist, und der Prozessor (32) ferner programmiert ist, um ein Beleuchtungs-Scanmuster in Antwort auf eine Eingabe durch die Anwenderschnittstelle zu ändern.

8. Chirurgisches System nach Anspruch 1, wobei der Prozessor (32) ferner programmiert ist, um einen Betriebszyklus der chirurgischen Laserquelle zu steuern, um ein ausgewähltes Spot-Muster für den chirurgischen Laserstrahl zu erzeugen.

9. Chirurgisches System nach Anspruch 8, wobei das chirurgische System (10) ferner eine Anwenderschnittstelle (50) aufweist und der Prozessor (32) ferner programmiert ist, um das gewählte Spot-Muster in Antwort auf eine Eingabe durch die Anwenderschnittstelle zu ändern.

10. Chirurgisches System nach Anspruch 1, wobei die chirurgische Laser-(40)-Quelle eine Mehrzahl an chirurgischen Laserstrahlen emittiert, und die Endosonde eine Mehrzahl an chirurgischen Laserfasern aufweist.

## Revendications

1. Système chirurgical (10), comprenant :
une source de laser chirurgical (40) utilisable pour émettre un laser chirurgical comprenant au moins un élément optique ;
un moteur de tomographie par cohérence optique (TCO) (30) utilisable pour émettre un faisceau TCO ;
une endosonde (100) couplée optiquement à la source de laser chirurgical (40) et au moteur de TCO (30), l'endosonde comprenant :
une fibre TCO (120) pour transmettre le faisceau TCO ;
une fibre laser chirurgicale (122) pour transmettre le faisceau laser chirurgical, le faisceau laser chirurgical passant à travers l'au moins un élément optique de la source de laser chirurgical ;
une canule (110) comprenant une première et une seconde canules internes contrarotatives (112, 114), respectivement, ayant une première optique de balayage (116) et une seconde optique de balayage, ladite canule renfermant la fibre TCO (120) et la fibre laser chirurgicale (122), couplées optiquement aux première et seconde optiques de balayage de telle sorte que le faisceau TCO et le faisceau laser chirurgical passent chacun à travers les première et seconde optiques de balayage, une contra-rotation des première et seconde optiques de balayage provoquant simultanément le balayage des deux du faisceau TCO et du faisceau laser ; et
un processeur (32) programmé pour commander le balayage par les optiques de balayage du faisceau TCO et du faisceau laser chirurgical sur une zone de tissu ciblée et pour détecter un signal de TCO provenant de la zone de tissu ciblée,
**caractérisé en ce que** l'au moins un élément optique est configuré pour produire de multiples taches à partir du faisceau laser chirurgical.

2. Système chirurgical selon la revendication 1, dans lequel le processeur (32) est programmé pour déterminer que la zone de tissu ciblée a été modifiée avec succès par le faisceau laser chirurgical et pour balayer en réponse le faisceau laser chirurgical et le faisceau TCO sur un autre tissu.

3. Système chirurgical selon la revendication 1, dans lequel le processeur (32) est programmé pour déterminer que la zone de tissu ciblée a été endommagée et pour lancer en réponse une action corrective.

4. Système chirurgical selon la revendication 3, dans lequel l'action corrective consiste à couper la source de laser chirurgical.

5. Système chirurgical selon la revendication 1, dans lequel le système chirurgical comprend en outre une source d'éclairage (20) couplée optiquement à l'endosonde (100), et l'endosonde comprend en outre une fibre d'éclairage (124).

6. Système chirurgical selon la revendication 5, dans lequel la fibre d'éclairage (124) est couplée optiquement à l'optique de balayage.

7. Système chirurgical selon la revendication 6, dans lequel le système chirurgical (10) comprend en outre une interface utilisateur (50), et le processeur (32) est en outre programmé pour changer un motif de balayage d'éclairage en réponse à une entrée provenant de l'interface utilisateur.

8. Système chirurgical selon la revendication 1, dans lequel le processeur (32) est en outre programmé pour contrôler un rapport cyclique de la source de laser chirurgical pour produire un motif de tache sélectionné pour le faisceau laser chirurgical.

9. Système chirurgical selon la revendication 8, dans lequel le système chirurgical (10) comprend en outre une interface utilisateur (50), et le processeur (32) est en outre programmé pour changer le motif de tache sélectionné en réponse à une entrée provenant de l'interface utilisateur.

10. Système chirurgical selon la revendication 1, dans lequel la source de laser chirurgical (40) émet une pluralité de faisceaux lasers chirurgicaux, et l'endosonde comprend une pluralité de fibres laser chirurgicales.
